(19) Europäisches Patentamt
European Patent Office
Office européen des brevets

(11) Veröffentlichungsnummer: **0 002 672**
**B1**

(12) **EUROPÄISCHE PATENTSCHRIFT**

(45) Veröffentlichungstag der Patentschrift: **26.08.81**

(21) Anmeldenummer: **78101509.4**

(22) Anmeldetag: **01.12.78**

(51) Int. Cl.³: **C 07 D 207/08,**
C 07 D 207/22,
C 07 D 207/14,
C 07 D 207/20,
A 61 K 31/40

(54) **Substituierte Pyrrolidine, Verfahren zu ihrer Herstellung und sie enthaltende Arzneimittel.**

(30) Priorität: **02.12.77 LU 78625**

(43) Veröffentlichungstag der Anmeldung:
**11.07.79 Patentblatt 79/14**

(45) Bekanntmachung des Hinweises auf die Patenterteilung:
**26.08.81 Patentblatt 81/34**

(84) Benannte Vertragsstaaten:
**BE CH DE FR IT LU NL SE**

(56) Entgegenhaltungen:
**JOURNAL OF MEDICINAL CHEMISTRY, Vol. 19, Nr. 10. Oktober 1976, Easton, Seiten 1195—1201**

(73) Patentinhaber: **Byk Gulden Lomberg Chemische Fabrik GmbH**
**Byk-Gulden-Strasse 2**
**D-7750 Konstanz (DE)**

(72) Erfinder: **Eistetter, Klaus, Dr.**
**Säntisblick 7**
**D-7750 Konstanz 19 (DE)**
Erfinder: **Kley, Hans-Peter, Dr.**
**Alemannenstrasse 6**
**D-7753 Allensbach (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 002 672 B1**

# Substituierte Pyrrolidine, Verfahren zu ihrer Herstellung und sie enthaltende Arzneimittel

Die Erfindung betrifft substituierte Pyrrolidine, Verfahren zu ihrer Herstellung, ihre Verwendung und sie enthaltende Arzneimittel.

2-Phenacyl-pyrrolidine werden von R. B. Herbert et al. (J.C.S. Chem. Comm. *1976*, 450—451) als Zwischenprodukte für Alkaloide von Septicin-Typ beschrieben. Aus den deutschen Offenlegungsschriften DE—OS 24 17 782 und DE—OS 24 18 480 sind 1-(aryl-substituiertes Phenyl)-2-pyrrolidin-2-yl-ethanone und -ethanole bekannt, die als Antikoagulantien verwendet werden sollen. Die Eigenschaften von bestimmten Pyrrolidin-2-yl-ethanonen und -ethanolen als Inhibitoren der Blutplättchenaggregation wurden von J. M. Grisar et al. [J. Med. Chem. 19 (1976) 1195—1201] untersucht. Es wurde nun gefunden, daß in 3-Stellung der Phenylgruppe durch spezielle Substituenten substituierte 1-Phenyl-2-pyrrolidin-2-yl-ethanole sowie ihre Ester und Ether unerwartete und vorteilhafte Eigenschaften aufweisen.

Gegenstand der Erfindung sind substituierte Pyrrolidine der allgemeinen Formel I

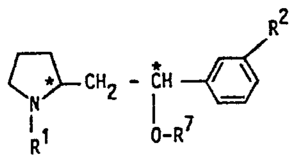

(I),

worin

$R^1$ ein Wasserstoffatom oder eine Alkylgruppe mit 1 bis 7 Kohlenstoffatomen,

$R^2$ ein Chloratom, eine Hydroxygruppe, eine Alkylgruppe mit 1 bis 4 Kohlenstoffatomen oder eine Alkoxygruppe mit 1 bis 4 Kohlenstoffatomen und

$R^7$ ein Wasserstoffatom, eine Alkanoylgruppe mit 1 bis 11 Kohlenstoffatomen, eine Alkylgruppe mit 1 bis 7 Kohlenstoffatomen oder eine Tetrahydropyranylgruppe bedeuten, und ihre Säureadditionssalze.

Als Alkylgruppen kommen geradkettige oder verzweigte Alkylreste in Betracht. Geradkettige Alkylreste mit 1 bis 7 Kohlenstoffatomen sind beispielsweise der Methyl-, Ethyl-, n-Butyl- und n-Heptylrest, von denen die mit 1 bis 3, insbesondere mit 1, Kohlenstoffatom(en) bevorzugt sind. Verzweigte Alkylreste mit 3 bis 7 Kohlenstoffatomen sind unter anderem der Isopropyl-, sek.-Butyl-, tert.-Butyl, tert.-Pentyl- und 2-Methylpentyl-rest, von denen die mit 3 und 4 Kohlenstoffatomen bevorzugt sind. Als Alkylreste von Alkoxygruppen kommen sowohl geradkettige als auch verzweigte Alkylreste in Frage.

Als Alkanoylgruppen mit 1 bis 11, vorzugsweise 2 bis 7 Kohlenstoffatomen, seien beispielsweise genannt die Heptanoyl-, Caproyl-, Pivaloyl-, Butyryl-, Isobutyryl- oder Propionylgruppe, insbesondere die Acetylgruppe.

Als Salze kommen alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze werden durch dem Fachmann bekannte Verfahren in pharmakologisch verträgliche Salze übergeführt. Als solche seien beispielsweise genannt wasserlösliche oder wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat [2-(4-Hydroxybenzoyl)-benzoat], Fendizoat (2-[(2′-Hydroxy-4-biphenylyl)-carbonyl]-benzoat), Propionat, Butyrat, Sulfosalicylat, Maleat, Laurat, Malat, Fumarat, Succinat, Oxalat, Tartrat, Amsonat (4,4′-Diaminostilben-2,2′-disulfonat), Embonat [4,4′-Methylen-bis-(3-hydroxy-2-naphthoat)], Metembonat [4,4′-Methylen-bis-(3-methoxy-2-naphthoat)], Stearat, Tosilat (p-Toluolsulfonat), 2-Hydroxy-3-naphthoat, 3-Hydroxy-2-naphthoat, Mesilat (Methansulfonat).

Eine Ausgestaltung der Erfindung sind substituierte Pyrrolidine der allegemeinen Formel I*

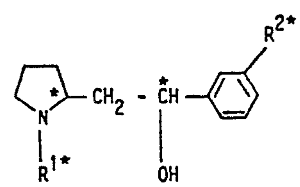

(I*),

worin

$R^{1*}$ ein Wasserstoffatom oder eine Alkylgruppe mit 1 bis 3 Kohlenstoffatomen und

$R^{2*}$ eine Hydroxygruppe, eine Methylgruppe oder eine Methoxygruppe bedeuten,

und ihre Säureadditionssalze.

Eine bevorzugte Ausgestaltung der Erfindung sind substituierte Pyrrolidine der allgemeinen Formel I**

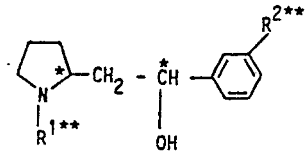

(I**),

worin
$R^{1**}$ ein Wasserstoffatom oder eine Methylgruppe und
$R^{2**}$ eine Methylgruppe oder eine Methoxygruppe bedeuten,
und ihre pharmakologisch verträglichen Säureadditionssalze.

Als Vertreter der erfindungsgemäßen Verbindungen seien beispielsweise
1-(3-Chlorphenyl)-2-pyrrolidin-2-yl-ethanol,
1-(3-Isopropoxyphenyl)-2-(1-methylpyrrolidin-2-yl)-ethanol,
1-(3-Ethylphenyl)-2-(1-ethylpyrrolidin-2-yl)-ethanol,
1-(3-Hydroxyphenyl)-2-(1-isopropylpyrrolidin-2-yl)-ethanol,
1-(3-n-Butoxyphenyl)-2-(1-n-heptylpyrrolidin-2-yl)-ethanol,
1-(3-tert.-Butylphenyl)-2-(1-methylpyrrolidin-2-yl)-ethanol,
1-(3-Chlorphenyl)-2-(1-methylpyrrolidin-2-yl)-ethanol,
Heptansäure-[1-(3-methoxyphenyl)-2-(1-methylpyrrolidin-2-yl)-ethyl]-ester,
Isobuttersäure-[1-(3-methoxyphenyl)-2-(1-methylpyrrolidin-2-yl)-ethyl]-ester,
Propionsäure-[1-(3-methoxyphenyl)-2-(1-isopropylpyrrolidin-2-yl)-ethyl]-ester,
2-[1-(3-Methoxyphenyl)-2-(1-isobutylpyrrolidin-2-yl)-ethoxy]-tetrahydropyran,
2-[2-Isopropoxy-2-(3-methoxyphenyl)-ethyl]-1-methylpyrrolidin,
2-[2-Hexyloxy-2-(3-methoxyphenyl)-ethyl]-1-methylpyrrolidin vorzugsweise
1-(3-Methoxyphenyl)-2-pyrrolidin-2-yl-ethanol,
2-(1-Methylpyrrolidin-2-yl)-1-(m-tolyl)-ethanol,
1-(3-Methoxyphenyl)-2-(1-methylpyrrolidin-2-yl)-ethanol,
und deren Säureadditionssalze genannt.

Die substituierten Pyrrolidine der allgemeinen Formel I bzw. der Ausgestaltungen I* und I** besitzen an den mit (*) gekennzeichneten Kohlenstoffatomen je ein Chiralitätszentrum. Die Erfindung schließt daher sowohl die Diastereomeren, die Racemate und die Enantiomeren als auch deren Gemische ein. Bei der Bezeichnung der Konfiguration der Stereomeren wird der Regel von R.S. Cahn, C. K. Ingold und V. Prelog [vgl. Angew. Chem. 78 (1966) 413 oder IUPAC Tentative rules for the nomenclature of organic chemistry, Sect. E. Fundamental Stereochemistry, J. org. Chem. 35 (1970) 2849 oder Chemical Abstracts stereochemical nomenclature of organic substances in the ninth collective period (1972—1976), J. Chem. Inf. Comput. Sci. 15 (1975) 67 gefolgt.

Die substituierten Pyrrolidine der allgemeinen Formel I bzw. der Ausgestaltungen I* und I** und die pharmakologisch, d.h. biologisch verträglichen Säureadditionssalze besitzen wertvolle Eigenschaften, die sie gewerblich verwertbar machen. Sie entfalten eine starke analgetische Wirkung. Ferner sind sie durch geringe Toxizität bei Abwesenheit wesentlicher Nebenwirkungen gekennzeichnet. Als weiterer Vorteil der erfindungsgemäßen Verbindungen its anzusehen, daß sie weder eine Abhängigkeit (Sucht) noch eine Toleranzentwicklung (Anpassung) bewirken.

Die ausgezeichnete und spezifische Wirksamkeit der substituierten Pyrrolidine ermöglicht ihren Einsatz sowohl in der Human- als auch in der Veterinärmedizin, wobei sie zur Prophylaxe vor Beschwerden oder besonders zur Behandlung bereits aufgetretener Symptome verwendet werden.

Als Indikationen für den humanmedizinischen Bereich seien z.B. beim Mann, bei der Frau oder bei Kindern akute oder chronische Schmerzzustände verschiedener Ätiologie, beispielsweise postoperative Schmerzen, Schmerzen bei Verletzungen, Verätzungen oder Verbrennungen, spastische Schmerzen (z.B. bei Koliken) im Gallen- oder Harnwegsbereich, chronische Schmerzen bei Knochen- und Gelenkerkrankungen und bei Neuraligien, Schmerzen bei diagnotischen und therapeutischen Eingriffen und nach Geburten, genannt.

Im veterinärmedizinischen Bereich kommen als Indikationen ähnliche Schmerzzustände in Betracht. Beispielsweise werden höhere Tiere wie Nutz- und Haustiere behandelt.

Gegenstand der Erfindung sind auch Arzneimittel, die substituierte Pyrrolidine der Formel I und/oder ihre pharmakologisch verträglichen Säureadditionssalze enthalten.

Bevorzugte Arzneimittel sind solche, die substituierte Pyrrolidine der Ausgestalung I*, insbesondere der Ausgestaltung I**, und/oder ihre pharmakologisch verträglichen Säureadditionssalze enthalten.

Die Arzneimittel werden nach an sich bekannten Verfahren hergestellt. Als Arzneimittel werden die erfindungsgemäßen Verbindungen entweder als solche oder vorzugsweise in Kombination mit geeigneten pharmazeutischen Trägerstoffen eingesetzt. Enthalten die neuen pharmazeutischen Zubereitungen neben den erfindungsgemäßen Verbindungen pharmazeutische Trägerstoffe, beträgt der

Wirkstoffgehalt dieser Mischungen 0,5 bis 95, vorzugsweise 15 bis 75, Gewichtsprozent der Gesamtmischung.

In Übereinstimmung mit der Erfindung werden im human- und veterinärmedizinischen Bereich die Wirkstoffe in jeder geeigneten Form angewandt unter der Voraussetzung, daß die Ausbildung bzw. Aufrechterhaltung von ausreichenden Wirkstoffspiegeln gewährleistet ist. Das kann beispielsweise durch orale, rektale oder parenterale (intravenöse, intramusculäre, subcutane) Gabe in geeigneten Dosen erreicht werden. Vorteilhafterweise liegt die pharmazeutische Zubereitung des Wirkstoffes in Form von Einheitsdosen vor, die auf die gewünscht Verabreichung abgestimmt sind. Eine Einheitsdosis kann zum Beispiel eine Tablette, ein Dragee, eine Kapsel oder eine gemessene Volumenmenge eines Pulvers, eines Granulates, einer Lösung, einer Emulsion oder einer Suspension oder eines Suppositoriums sein.

Unter "Einheitsdosis" im Sinne der vorliegenden Erfindung wird eine physikalisch bestimmte Einheit, die eine individuelle Menge des aktiven Bestandteils in Kombination mit einem pharmazeutischen Trägerstoff enthält, verstanden, deren Wirkstoffgehalt einem Bruchteil oder Vielfachen einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel, der Einheitsdosis benötigt wird, ist die Einheitsdosis vorteilhafterweise teilbar, z.B. in Form einer Tablette mit Bruchkerbe.

Die pharmazeutischen Zubereitungen gemäß der Erfindung enthalten, wenn sie in Einheitsdosen vorliegen und für die Applikation z.B. am Menschen bestimmt sind, etwa, 0,1 bis 250 mg, vorteilhafterweise 1,0 bis 100 mg und insbesondere 5 bis 50 mg Wirkstoff.

Im allgemeinen hat es sich in der Humanmedizin als zweckmäßig erwiesen, den oder die Wirkstoffe bei oraler Gabe in einer Tagesdosis von etwa 0,01 bis etwa 5, vorzugsweise 0,1 bis 3 mg/kg Körpergewicht, gegebenenfalls in Form mehrerer, vorzugsweise 1 bis 3 Einzelgaben zur Erzielung der gewünschten Ergebnisse zu verabreichen. Eine Einzelgabe enthält den oder die Wirkstoffe in Mengen von etwa 0,01 bis 2,5, vorzugsweise 0,1 bis 1,5 mg/kg Körpergewicht. Bei einer parenteralen, z.B. intravenösen Behandlung kommen entsprechende Dosierungen zur Anwendung.

Die therapeutische Verabreichung der pharmazeutischen Zubereitung erfolgt 1 bis 4 mal am Tag zu festgelegten oder variierenden Zeitpunkten, z.B. jeweils nach den Mahlzeiten und/oder am Abend. Es kann jedoch erforderlich sein, von den genannten Dosierungen abzuweichen, und zwar in Abhängigkeit von der Art, dem Körpergewicht und dem Alter des zu behandelnden Objektes, der Art und der Schwere der Erkrankung, der Art der Zubereitung und der Applikation der Arzneimittles sowie dem Zeitraum bzw. Intervall, innerhalb welchem die Verabreichung erfolgt. So kann es bei chronischen Schmerzzuständen ausreichend sein, mit geringeren Mengen Wirkstoff auszukommen, während in schweren Fällen akuter Schmerzzustände die oben angeführten Wirkstoffmengen ohne Bedenken kurzfristig überschritten werden können.

Die Festlegung der jeweils erforderlichen optimalen Dosierung und Applikationsart der Wirkstoffe erfolgt durch den Fachmann aufgrund seines Fachwissens.

Die pharmazeutischen Zubereitungen bestehen bevorzugt aus den erfindungsgemäßen Wirkstoffen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses, für den therapeutisch aktiven Bestandteil in Anwendung kommen. Ein trägerstoff kann z.B. als Vermittler für die Arzneimittelaufnahme durch den Körper, als Formulierungshilfsmittel, als Süßungsmittel, als Geschmackskorrigenz, als Farbstoff oder als Konservierungsmittel dienen.

Zur oralen Anwendung kommen z.B. Tabletten, Dragees, harte und weiche Kapseln, z.B. aus Gelatine, dispergierbare Pulver, Granulate, wäßrige und ölige Suspensionen, Emulsionen, Lösungen oder Sirupe.

Tabletten enthalten inerte Verdünnungsmittel, z.B. Calciumcarbonat, Calciumphosphat, Natriumphosphat oder Lactose; Granulierungs- und Verteilungsmittel, z.B. Maisstärke oder Alginate; Bindemittel, z.B. Stärke, Gelatine oder Akaziengummi; und Gleitmittel, z.B. Aluminium- oder Magnesiumstearat, Talkum oder Silikonöl. Gewünschtenfalls werden sie zusätzlich mit einem Überzug versehen, der auch so beschaffen sein kann, daß er eine verzögerte Auflösung und Resorption des Arzneimittels im Gastrointestinaltrakt bewirkt und damit z.B. eine bessere Verträglichkeit, Prothrahierung oder eine Retardierung erreicht wird. Gelatinekapseln enthalten den Arzneistoff im allgemeinen vermischt mit einem Verdünnungsmittel, z.B. einem festen Verdünnungsmittel, wie Calciumcarbonat oder Kaolin, oder einem öligen Verdünnungsmittel, wie Neutral-, Oliven-, Erdnuß- oder Paraffinöl.

Wäßrige Suspensionen enthalten gegebenenfalls Suspendiermittel, z.B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z.B. Polyoxyethylenstearat, Heptadecaethylenoxycetanol, Polyoxyethylensorbitolmonooleat, Polyoxyethylensorbitanmonooleat oder Lecithin; Konservierungsmittel, z.B. Methyl- oder Propylhydroxybenzoate; Geschmacksmittel; Süßungsmittel, z.B. Saccharose, Lactose, Natriumcyclamat, Dextrose, Invertzuckersirup.

Ölige Suspensionen enthalten z.B. Erdnuß-, Oliven-, Sesam-, Kokos- oder Paraffinöl und Verdickungsmittel, wie z.B. Bienenwachs, Hartparaffin oder Cetylalkohol; ferner Süßungsmittel, Geschmacksmittel und Antioxidantien.

Emulsionen enthalten z.B. Oliven-, Erdnuß- oder Paraffinöl neben Emulgiermitteln, wie Akaziengummi, Traganthgummi, Phosphatiden, Sorbitanmonooleat, Polyoxyethylensorbitanmonooleat, und Süßungs- und Geschmacksmitteln.

Zur rektalen Anwendung der Arzneistoffe werden Suppositorien verwendet, die mit Hilfe von bei Rektaltemperatur schmelzenden Bindemitteln, beispielsweise Kakaobutter oder Polyethylenglykol, hergestellt werden.

Zur parenteralen Anwendung der Arzneistoffe dienen steril injizierbare wäßrige Suspensionen, isotonische Salzlösungen oder sonstige Lösungen, die Dispergier- oder Benetzungsmittel und/oder pharmakologisch verträgliche Verdünnungsmittel, z.B. Propylen- oder Butylenglykol, enthalten.

Neben den neuen erfindungsgemäßen Verbindungen können die pharmazeutischen Zubereitungen auch einen oder mehrere pharmakologisch aktive Bestandteile aus anderen Arzneimittelgruppen enthalten. Beispielsweise seien genannt Antipyretika, Analgetika, Antiphlogistika, z.B. Phenylbutazon, Bumadizon, Phenacetin, Indometazin, Lonazolac; Hypnotika, wie Barbiturate, z.B. Hexobarbital, Vinylbarbital; Stimulantien, wie Coffein; Sedativa, wie Meprobamat und Benzodiazepine, z.B. Diazepam, Oxazepam, Chlordiazepoxid; Spasmolytika, wie Papaverin; Vitamine, wie Vitamin $B_1$, Vitamin $B_6$, Vitamin $B_{12}$, Vitamin C, z.B. Vitamin $B_1$-chloridhydrochlorid, Vitamin $B_6$-hydrochlorid, Cyanocobalamin.

Gegenstand der Erfindung ist ferner eine Verfahren zur Herstellung der substituierten Pyrrolidine der allgemeinen Formel I bzw. der Ausgestaltungen I* und I** sowie der Säureadditionssalze, das dadurch gekennzeichnet ist, daß man ein Keton der allgemeinen Formel II oder III

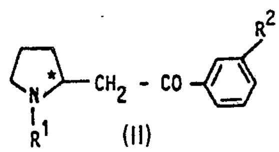
(II)

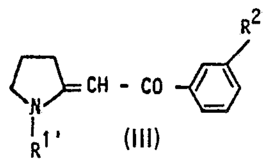
(III)

worin

$R^1$ und $R^2$ die oben angegebene Bedeutung haben und $R^{1'}$ eine Alkylgruppe mit 1 bis 7 Kohlenstoffatomen bedeutet, oder dessen Säureadditionssalz reduziert und gegebenenfalls anschließend acyliert oder verethert und/oder die erhaltene freie Base oder ihr Säureadditionssalz ineinander überführt.

Die Reduktion der Ketone der allgemeinen Formel II und III erfolgt nach dem Fachmann an sich bekannten. Verfahren. Werden Ausgangsverbindungen III eingesetzt, so kann die Reduktion ein- oder zweistufig durchgeführt werden. Wegen der leichteren Versuchsführung und der günstigeren Ausbeuten ist das zweistufige gegenüber dem einstufigen Verfahren bevorzugt.

Für die Reduktion der Ketogruppe in den Ausgangsverbindungen II geeignete Reduktionsmittel sind z.B. reduzierende komplexe Metallhydride, wie Lithiumhydridoaluminat (= Lithiumalumininiumhydrid) in Ethern, wie Diethylether, Tetrahydrofuran, oder Natriumhydridoborat (= Natriumborhydrid) in Alkoholen, wie Methanol, Ethanol, Isopropanol und deren Mischungen untereinander sowie mit Wasser. Bevorzugt wird die Reduktion mit Natriumhydridoborat durchgeführt. Die Umsetzung erfolgt bei Temperaturen zwischen 0 und 80°C bei Reaktionszeiten von 10 Minuten bis 6 Stunden. Zweckmäßigerweise wird die Reaktion bei Temperaturen um 0°C in geeigneten Lösungsmitteln durch portionsweise Zugabe des Reduktionsmittels durchgeführt, wobei die Temperatur der Lösung allmählich auf Raumtemperatur ansteigt. Nach vollständiger Umsetzung werden die Reaktionsgemische durch Behandlung mit Wasser in üblicher Weise aufgearbeitet. Die Reduktion erfolgt alternativ mit Wasserstoff in Gegenwart von Katalysatoren, wie Raney-Nickel, Platin, Platin auf Aktivkohle etc., bei 0 bis 80°C und Drücken von 1—500 bar.

Bei zweistufiger Reaktionsführung erfolgt die Reduktion der Ausgangsverbindungen III zunächst zu den Verbindungen II, wobei als Reduktionsmittel beispielsweise Lithiumhydridoaluminat oder Natriumdihydrido-bis-(2-methoxyethoxy)-aluminat eingesetzt werden. Die Umsetzung erfolgt in den üblichen Lösungsmitteln, wie Ethern, z.B. Diethylether, Tetrahydrofuran, oder Kohlenwasserstoffen, z.B. Benzol, Toluol, bei —20 bis 60°C, bevorzugt zwischen 0°C und Raumtemperatur, bei Reaktionszeiten von 10 Minuten bis 10 Stunden. Bevorzugt wird die Durchführung der Reaktion in Tetrahydrofuran bei Temperaturen um 0°C unter portionsweiser Zugabe von Lithiumaluminiumhydrid, wobei die Reaktion im allgemeinen nach einer halben Stunde beendet ist. Weitere Reduktion der erhaltenen Verbindungen II erfolgt dann nach dem vorstehend beschriebenen Verfahren.

Bei einstufiger Reaktionsführung erfolgt die Reduktion der Ausgangsverbindungen III mit Lithiumhydridoaluminat oder Natriumdihydrido-bis-(2-methoxyethoxy)-aluminat in Ethern, wie Diethylether, Tetrahydrofuran, oder mit Natriumborhydrid in Alkoholen, wie Methanol, Ethanol, Propanol, oder Alkohol-Wasser-Gemischen bei Temperaturen von 20°C bis zur Siedetemperatur des

Lösungsmittels, wobei das Reduktionsmittel im Überschuß eingesetzt wird. Die Reaktionszeiten betragen 1 bis 48 Stunden.

Eine gegebenenfalls sich anschließende Acylierung der Hydroxygruppe wird nach dem Fachmann bekannten Methoden, z.B. durch Umsetzung mit den entsprechenden Säureanhydriden bzw. -halogeniden, gegebenenfalls in Gegenwart eines Protonenacceptors, z.B. Alkalimetallcarbonats oder tert. Amins, wie Pyridin, Triethylamin, durchgeführt. Die Reaktion kann auch in einem inerten Lösungsmittel, z.B. Benzol, Cyclohexan, Diethylether, Methylenchlorid, ausgeführt werden. Die sich gegebenenfalls anschließende Veretherung erfolgt beispielsweise durch Umsetzung mit Alkylhalogeniden, z.B. Alkyliodiden, in inerten Lösungsmitteln, z.B. Dimethylformamid, Toluol Tetrahydrofuran, in Gegenwart einer starken Base, z.B. Kaliumhydroxid oder Natriumhydrid. Tetrahydropyranylether erhält man durch Umsetzung mit Dihydropyran unter Säurekatalyse, z.B. in Gegenwart von Sulfonsäuren, beispielsweise p-Toluolsulfonsäure.

Säureanlagerungssalze erhält man durch Auflösen der freien Base in einem geeigneten Lösungsmittel, z.B. Aceton, Wasser, einem niedermolekularen aliphatischen Alkohol (Ethanol, Isopropanol), das die gewünschte Säure enthält, oder dem die gewünschte Säure anschließend zugegeben wird. Die Salze werden durch Filtrieren, Ausfällen mit einem Nichtlösungsmittel für das Anlagerungssalz oder durch Verdampfen des Lösungsmittels gewonnen.

Erhaltene Salze, z.B. die Hydrochloride, können durch Neutralisation mit wäßrigem Natrium- oder Kaliumhydroxid in die freie Base umgewandelt werden, die dann durch Lösungsmittelextraktion mit einem geeigneten, nicht mit Wasser mischbaren Lösungsmittel, wie Chloroform, Dichlormethan, Diethylether, Benzol, Toluol, Cyclohexan etc., gewonnen wird. Die freien Basen können auch durch Neutralisation eines Säureanlagerungssalzes mit Natriummethylat in Methanol und Isolierung der Base nach bekannten Verfahren gewonnen werden.

Diastereomere und Racemate werden nach üblichen Verfahren getrennt. Die Trennung der Diastereomeren erfolgt z.B. aufgrund ihrer unterschiedlichen physikalisch-chemischen Eigenschaften wie Schmelzpunkt, Löslichkeit; die Trennung der Racemate in die optisch-aktiven Isomeren erfolgt mit Hilfe optisch-aktiver Spaltungsmittel, z.B. optisch-aktiver Säuren, wie Weinsäure, Dibenzoylweinsäure, Camphersulfonsäure, Desoxycholsäure. Man kann racemische Gemische auch durch Chromatographie über optisch-aktive Sorptionsmittel in die optischen Isomeren auftrennen.

Die als Ausgangsverbindungen einzusetzenden Ethanone der allgemeinen Formel II werden nach an sich bekannten Verfahren erhalten. Beispielsweise werden Ethanone II, in denen $R^1$ ein Wasserstoffatom bedeutet, durch Kondensation von Pyrrolin-1 mit entsprechenden 3-substituierten Acetophenonen und Methylmagnesiumcarbonat nach dem von J.M. Grisar et al. [Synthesis 1974, 284; J. Med. Chem. 19 (1976) 1195] beschriebenen Verfahren hergestellt; sie werden durch N-Alkylierung in Ethanone II, in denen $R^1$ eine Alkylgruppe mit 1 bis 7 Kohlenstoffatomen bedeutet, übergeführt. Die N-Alkylierung erfolgt mit dem Fachmann bekannten Alkylierungsmitteln, wie Alkylhalogeniden, z.B. Ethyliodid, Alkylsulfonaten z.B. Methyltosylat, Alkylsulfaten, z.B. Dimethylsulfat, in inerten Lösungsmitteln, wie Ketonen, z.B. Aceton, Ethylmethylketon, Alkoholen, z.B. Methanol, Ethanol, Isopropanol, oder ohne Lösungsmittel unter Zusatz einer Hilfsbase, wie Natriumcarbonat, Kaliumcarbonat, Pyridin, Triethylamin.

Die Ausgangsverbindungen der allgemeinen Formel III lassen sich nach verschiedenen Verfahren herstellen. Beispielsweise werden sie durch Umsetzung von 1-Alkyl-2-alkoxy-pyrrolidinen der allgemeinen Formel IV mit entsprechenden 3-substituierten Acetophenonen V

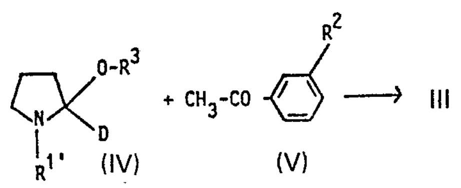

(IV) (V)

worin

$R^{1'}$ und $R^2$ die oben angegebene Bedeutung haben,

D eine —O—$R^4$-Gruppe oder eine —$NR^5(R^6)$-Gruppe bedeutet,

$R^3$, $R^4$, $R^5$ oder $R^6$ gleich oder verschieden sind und eine Alkylgruppe mit 1 bis 5 Kohlenstoffatomen, vorzugsweise eine Ethylgruppe oder Methylgruppe, bedeuten, erhalten. Die Umsetzung erfolgt in Abwesenheit oder Gegenwart basischer Katalysatoren, wie Triethylamin, 1,5-Diazabicyclo[5.4.0]undec-5-en, bei Temperaturen zwischen Raumtemperatur und 100°C, ohne Lösungsmittel oder in inerten Lösungsmitteln, wie Benzol, Toluol, Methylenchlorid.

Die Ausgangsverbindungen der allgemeinen Formel III werden alternativ durch Umsetzung von Pyrroliniumsalzen VI mit entsprechenden 3-substituierten Acetophenonen V

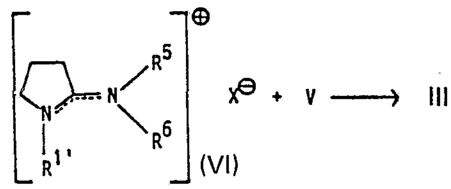

(VI)

worin

$R^{1'}$, $R^5$ und $R^6$ die oben angegebene Bedeutung haben und $X^{\ominus}$ für ein Äquivalent eines Anions einer organischen oder anorganischen Säure steht, erhalten. Die Umsetzung erfolgt in inerten Lösungsmitteln, wie Alkoholen, z.B. Methanol, Ethanol, Isopropanol, Butanolen, Pentanolen, in Gegenwart starker Basen, wie Alkalimetallalkoholaten, z.B. Natriummethanolat, Natriumethanolat, Kaliumpropanolat, Kalium-tert.-butanolat, Kalium-tert.-pentanolat, bei Temperaturen von 20 bis 150°C, vorzugsweise 80 bis 100°C.

Die 1-Alkyl-2-alkoxy-pyrrolidine IV werden erhalten, indem man Salze der Formel VI mit Alkalimetallalkoholaten, wie Natriummethanolat, -ethanolat etc. in geeigneten Lösungsmitteln umsetzt [in Anlehnung an H. Bredereck et al. Chem.Ber.97(1964)3081, Chem.Ber.98(1965)1078]. Bevorzugt Lösungsmittel bei der Herstellung von Pyrrolidinen IV, in denen D eine —O—$R^4$-Gruppe bedeutet, sind Alkohole $R^4$—OH, in denen $R^4$ die oben angegebene Bedeutung hat bevorzugte Lösungsmittel bei der Herstellung von Pyrrolidinen IV, in denen D eine —$NR^5(R^6)$-Gruppe bedeutet, sind inerte Lösungsmittel, wie Benzol und Ether, z.B. Diethylether.

Die Herstellung der Salze VI erfolgt in Analogie zu H. Bredereck et al. [Chem.Ber.97(1964) 3081] durch Umsetzung der entsprechenden N-substituierten 2-Pyrrolidinone mit Alkylierungsmitteln, wie Diethylsulfat, Methyliodid, bevorzugt Dimethylsulfat, in inerten Lösungsmitteln, bei Raumtemperatur bis 120°C, bevorzugt ohne Lösungsmittel bei Temperaturen um 80°C und anschließende Reaktion mit Aminen $HNR^5(R^6)$, in denen $R^5$ und $R^6$ die oben angegebene Bedeutung haben, oder durch Umsetzung der entsprechenden N-substituierten Pyrrolidinone mit anorganischen Säurechloriden, wie Phosphoroxidtrichlorid, Phosgen, und anschließende Reaktion mit Aminen $HNR^5(R^6)$, in denen $R^5$ und $R^6$ die oben angegebene Bedeutung haben, in inerten Lösungsmitteln, wie Benzol, bei Temperaturen zwischen 0 und 100°C, bevorzugt 20 bis 60°C, oder ohne Lösungsmittel bei Temperaturen zwischen 0 und 100°C, bevorzugt 40 bis 80°C.

Die folgenden Beispiele erläutern die Erfindung näher, ohne sie einzuschränken. Schmp. bedeutet Schmelzpunkt, Kp. bedeutet Siedepunkt, Temperaturangaben erfolgen in °C.

## Beispiele

### Beispiel 1

*2-Dimethylamino-1-methyl-1-pyrroliniummethylsulfat*

Man tropft unter Rühren zu einer Lösung von 6,76 g Dimethylamin in 45 ml Benzol 22,5 g 2-Methoxy-1-methyl-1-pyrroliniummethylsulfat und kocht anschließend 1 Stunde unter Rückfluß. Die schwere Phase wird abgetrennt, zweimal mit Diethylether ausgeschüttelt und im Vakuum von Lösungsmittelresten befreit. Man erhält 19,4 g (81,4% der Theorie) rotbraunes Öl.

### Beispiel 2

*1-Isopropyl-2-methoxy-1-pyrroliniummethylsulfat*

89,6 g 1-Isopropylpyrrolidinon-2 und 88,9 g Dimethylsulfat werden 3 Stunden bei 80° gerührt. Man extrahiert das Reaktionsgemisch 5 mal mit je 50 ml Ether und trocknet das rote Öl im Hochvakuum. Man erhält 167,2 g (94% der Theorie).

Dünnschichtchromatographie: Schicht Kieselgel neutral, Laufmittel Chloroform/Methanol 9:1 $R_F$ — Wert 0,30, Färbereagenz: Ioddampf.

### Beispiel 3

*1-Isopropyl-2-dimethylamino-1-pyrroliniummethylsulfat*

161 g 1-Isopropyl-2-methoxy-1-pyrrolinium-methylsulfat werden unter Rühren zu einer Lösung von 43,8 g Dimethylamin in 283 ml Benzol getropft. Anschließend kocht man 1,5 Stunden unter Rückfluß, trennt die schwere Phase ab und wäscht sie 4 mal mit je 50 ml Diethylether. Man erhält nach dem Trocknen im Hochvakuum 154 g (91% der Theorie) rötliches Öl.

### Beispiel 4

*1-(3-Methoxyphenyl)-2-(1-methyl-2-pyrrolidinyliden)-ethanon*

Zu einer Mischung aus 107 g 2-Dimethylamino-1-methyl-1-pyrroliniummethylsulfat und 45,1 g 3-Methoxyacetophenon tropft man bei 90° unter Rühren eine Lösung von 10,35 g Natrium in 225 ml Ethanol während einer Stunde zu, rührt noch 2 Stunden bei dieser Temperatur und destilliert das Lösungsmittel im Vakuum weitgehend ab. Der abgekühlt Rückstand wird mit 300 ml Wasser und 300

ml Diethylether versetzt und gut durchgeschüttelt. Die Etherphase wird gesammelt, über Natriumsulfat getrocknet und eingeengt. Der kristalline Rückstand wird aus Chloroform/Cyclohexan umkristallisiert. Ausbeute 41,8 g gelbliche Kristalle vom Schmp. 92—93°.

Beispiel 5

*2-(1-Isopropyl-2-pyrrolidinyliden)-1-(3-methoxyphenyl)-ethanon*

Zu einer Mischung aus 88 g 1-Isopropyl-2-dimethylamino-1-pyrroliniummethylsulfat und 42 g 3-Methoxyacetophenon tropft man unter Rühren bei 90° innerhalb 2 Stunden eine Lösung von 7,6 Natrium in 100 ml Ethanol zu. Man rührt noch 4 Stunden bei dieser Temperatur, destilliert das Lösungsmittel im Vakuum ab und verteilt den Rückstand in 250 ml Dichlormethan und 250 ml Wasser. Die organische Phase wird gesammelt, über Natriumsulfat getrocknet und eingeengt. Das zurückbleibende Öl wird durch Erwärmen im Hochvakuum von überschüssigem 3-Methoxyacetophenon befreit. Ausbeute 42,8 g (59% der Theorie) braunes Öl.

Beispiel 6

*1-(3-Methylphenyl)-2-(1-methyl-2-pyrrolidinyliden)-ethanon*

Nach der in Beispiel 4 beschriebenen Arbeitsweise erhält man aus 100 g 3-Methylacetophenon 238,3 g 2-Dimethylamino-1-methyl-1-pyrroliniummethylsulfat und einer Lösung von 23 g Natrium in 500 ml Ethanol 84,7 g (53% der Theorie) der Titelverbindung vom Schmp. 60—62° (aus Cyclohexan).

Beispiel 7

*1-(3-Chlorphenyl)-2-(1-methyl-2-pyrrolidinyliden)-ethanon*

Nach der in Beispiel 4 beschriebenen Arbeitsweise erhält man aus 50 g 3-Chloracetophenon, 103 g 2-Dimethylamino-1-methyl-1-pyrroliniummethylsulfat und einer Lösung von 9,9 g Natrium in 130 ml Ethanol 39 g (51% der Theorie) der Titelverbindung als braunes Öl.

Beispiel 8

*1-(3-Methoxyphenyl)-2-(1-methyl-2-pyrrolidinyl)-ethanon*

Zu 144,8 g 1-(3-Methoxyphenyl)-2-(1-methyl-2-pyrrolidinyliden)-ethanon, gelöst in 1,4 Liter Tetrahydrofuran, gibt man bei 0° unter Rühren portionsweise 11,8 g Lithiumaluminiumhydrid. 30 Minuten nach beendeter Zugabe werden 300 ml Wasser vorsichtig unter Kühlung zugetropft, dann 800 ml Diethylether zugegeben und die organische Phase gesammelt. Man trocknet die organische Phase über Natriumsulfat, entfernt das Lösungsmittel durch Eindampfen, destilliert den öligen Rückstand im Vakuum und erhält 127 g (87% der Theorie) der Titelverbindung vom Kp. 115° bei 1,33 Pa., Pikrat (aus Ethanol): Schmp. 153—156°.

Beispiel 9

*1-(3-Methylphenyl)-2-(1-methyl-2-pyrrolidinyl)-ethanon*

Zu 66 g 1-(3-Methylphenyl)-2-(1-methyl-2-pyrrolidinyliden)-ethanon, gelöst in 550 ml Tetrahydrofuran, gibt man bei 0° portionsweise 5,8 g Lithiumaluminiumhydrid. 20 Minuten nach beendeter Zugabe wird tropfenweise so lange Wasser zugegeben, bis keine Gasentwicklung mehr beobachtet wird. Man dekantiert die hellgelbe organische Phase von dem gebildeten Niederschlag ab, extrahiert diesen mit Diethylether, trocknet die vereinigten organischen Phasen über Natriumsulfat und engt zu einem Öl ein. Nach der Destillation im Vakuum erhält man 61,7 g (92% der Theorie) der Titelverbindung vom Kp. 116° bei 1,33 pa. Pikrat (aus Ethanol): Schmp. 146—149°.

Beispiel 10

*2-(1-Isopropyl-2-pyrrolidinyl)-1-(3-methoxyphenyl)-ethanon*

Nach der in Beispiel 8 beschriebenen Arbeitsweise erhält man aus 38,8 g 2-(1-Isopropyl-2-pyrrolidinyliden)-1-(3-methoxyphenyl)-ethanon 24,8 g (64% der Theorie) der Titelverbindung vom Kp. 120—123° bei 1,33 Pa.

Beispiel 11

*1-(3-Methoxyphenyl)-2-(1-methyl-2-pyrrolidinyl)-ethanol*

50 g 1-(3-Methoxyphenyl)-2-(1-methyl-2-pyrrolidinyl)-ethanon werden in 100 ml Methanol und 20 ml Wasser gelöst und danach unter Rühren portionsweise mit 4,05 g Natriumborhydrid versetzt. Man rührt eine Stunde nach, destilliert Methanol weitgehend ab, gibt 100 ml Wasser zu und extrahiert mit Methylenchlorid. Die organische Phase wird mit 400 ml 10%iger Salzsäure extrahiert, die salzsaure wässerige Phase wird mit Natronlauge alkalisiert und die freigesetzte Base wird mit Methylenchlorid extrahiert. Die organische Phase wird eingeengt und der ölige Rückstand destilliert. Man erhält 47,4 g (94% der Theorie) der Titelverbindung als Diastereomerengemisch vom Kp. 128° bei 0,133 Pa.

Beispiel 12

*(R*,S*)-1-(3-Methoxyphenyl)-2-(1-methyl-2-pyrrolidinyl)-ethanol*

45 g des Diastereomerengemisches von Beispiel 11 werden in 100 ml Cyclohexan in der Wärme gelöst und mit 100 ml tiefsiedendem Petrolether versetzt. Man läßt über Nacht bei 0° stehen und filtriert die abgeschiedenen Kristalle (21 g) ab. Nach dem Umkristallisieren aus 100 ml Cyclohexan/Petrolether (1:1) erhält man 14,4 g der Titelverbindung vom Schmp. 79—82°.

Durch Umsetzung der Base mit der äquivalenten Menge Säure erhält man die folgenden Salze:

Maleinat, farbloses Öl
Fumarat, farbloses Öl
Benzoat, farbloses Öl
Oxalat, farbloses Öl
Embonat, gelbes Öl

Beispiel 13

*(R*,R*)-1-(3-Methoxyphenyl)-2-(1-methyl-2-pyrrolidinyl)-ethanol*

Das in Beispiel 12 erhaltene Filtrat wird zu einem farblosen Öl eingeengt. Man löst das Öl in 50 ml Methanol und versetzt langsam mit etherischer Salzsäure, wobei das Hydrochlorid der Titelverbindung ausfällt, das aus Methanol/Diethylether umkristallisiert wird. Man erhält 12,1 g vom Schmp. 136—139°.

Beispiel 14

*2-(1-Isopropyl-2-pyrrolidinyl)-1-(3-methoxyphenyl)-ethanol*

Nach der in Beispiel 11 beschriebenen Arbeitsweise erhält man aus 23,6 g 2-(1-Isopropyl-2-pyrrolidinyl)-1-(3-methoxyphenyl)-ethanon und 3,5 g Natriumborhydrid 15,9 g der Titelverbindung als Diastereomerengemisch vom Kp. 130—132° bei 4 Pa.

Beispiel 15

*1-(3-Methylphenyl)-2-(1-methyl-2-pyrrolidinyl)-ethanol*

Nach der in Beispiel 11 beschriebenen Arbeitsweise erhält man aus 30 g 1-(3-Methylphenyl)-2-(1-methyl-2-pyrrolidinyl)-ethanon und 5,24 g Natriumborhydrid 23,4 g (77% der Theorie) der Titelverbindung als Diastereomerengemisch vom Kp. 105° bei 1,1 Pa, das bald teilweise kristallisiert.

Beispiel 16

*(R*,S*)-1-(3-Methylphenyl)-2-(1-methyl-2-pyrrolidinyl)-ethanol*

17 g des Diastereomerengemisches von Beispiel 15 werden aus 60 ml Petrolether (Kp. 50—70°) umkristallisiert. Das so erhaltene Rohprodukt wird erneut aus Petrolether umkristallisiert. Man erhält 3,5 g der Titelverbindung vom Schmp. 78—80°.

Beispiel 17

*(R*,R*)-1-(3-Methylphenyl)-2-(1-methyl-2-pyrrolidinyl)-ethanol*

Das in Beispiel 16 erhaltene erste Filtrat wird zu einem farblosen Öl eingeengt, das im Hochvakuum destilliert wird. Man erhält 6,0 g der Titelverbindung als farbloses Öl vom Kp. 105° bei 1,1 Pa.

Beispiel 18

*1-(3-Hydroxyphenyl)-2-(1-methyl-2-pyrrolidinyl)-ethanon*

40,5 g 1-(3-Methoxyphenyl)-2-(1-methyl-2-pyrrolidinyl)-ethanon werden mit 180 ml Essigsäure und 180 ml 47%iger Bromwasserstoffsäure 6 Stunden unter Rückfluß gekocht. Nach Einengen der Lösung wird der ölige Rückstand durch Behandeln mit 150 ml heißem Ethanol zur Kristallisation gebracht. Man läßt abkühlen und filtriert das Hydrobromid der Titelverbindung ab. Ausbeute 39,8 g (76% der Theorie) grünliche Kristalle vom Schmp. 165—168°.

Beispiel 19

*1-(3-Hydroxyphenyl)-2-(1-methyl-2-pyrrolidinyl)-ethanol*

Nach der in Beispiel 11 beschriebenen Arbeitsweise erhält man aus 4,5 g 1-(3-Hydroxyphenyl)-2-(1-methyl-2-pyrrolidinyl)-ethanon-hydrobromid und 1,0 g Natriumborhydrid 3,0 g der Titelverbindung als Diastereomerengemisch in Form eines gelben, zähen Öls, aus dem sich durch Umkristallisation aus Ethanol ein kristallines Diastereomer von Schmp. 147—150° isolieren läßt.

Beispiel 20

*1-(3-Methoxyphenyl)-2-(2-pyrrolidinyl)-ethanon*

60 ml einer 2-molaren Lösung von Methylmagnesiumcarbonat in Dimethylformamid werden im Kohlendioxid-Strom auf 120° erhitzt. Man gibt 5 g 3-Methoxyacetophenon zu, rührt unter Stickstoff 4

Stunden bei dieser Temperatur, läßt abkühlen und gibt 2,5 g 3,4-Dihydro-2H-pyrrol zu. Man rührt 2 Tage im Kohlendioxid-Strom, gießt auf eine Mischung aus 25 ml konzentrierter Salzsäure und 150 g Eis und extrahiert mit Dichlormethan. Nach dem Trocknen über Natriumsulfat engt man die organische Phase ein und erhält das Hydrochlorid der Titelverbindung (7,5 g) als gelbes Öl, das mit 150 ml Diethylether ca. 30 Minuten verrührt wird. Die ausgeschiedenen Kristalle werden aus 200 ml Methanol/Diethylether (1:4) umkristallisiert. Man erhält farblose Kristalle vom Schmp. 140—142.°.

Beispiel 21

*2-(1-n-Hexyl-2-pyrrolidinyl)-1-(3-methoxyphenyl)-ethanon*

5,11 g 1-(3-Methoxyphenyl)-2-(2-pyrrolidinyl)-ethanon-hydrochlorid, 5,6 g wasserfreies Kaliumcarbonat, 3,7 g 1-Bromhexan und 60 ml Ethyl-1-methylketon werden 18 Stunden unter Rückfluß gekocht. Man destilliert das Lösungsmittel ab, nimmt den Rückstand mit 50 ml Wasser auf und extrahiert 2 mal mit je 50 ml Diethylether. Die vereinigten organischen Phasen werden nach dem Trocknen über Natriumsulfat zu einem Öl eingeengt, das im Hochvakuum destilliert wird. Man erhält 5,0 g (82% der Theorie) der Titelverbindung vom Kp. 138—144° bei 0,66 Pa.

Beispiel 22

*2-(1-n-Hexyl-2-pyrrolidinyl)-1-(3-methoxyphenyl)-ethanol*

Nach der in Beispiel 11 beschriebenen Arbeitsweise erhält man aus 4,0 g 2-(1-n-Hexyl-2-pyrrolidinyl)-1-(3-methoxyphenyl)-ethanon und 0,5 g Natriumborhydrid 3,5 g (87% der Theorie) der Titelverbindung als öliges Diastereomerengemisch vom Kp. 138—140° bei 0,66 Pa.

Beispiel 23

*1-(3-Chlorphenyl)-2-(1-methyl-2-pyrrolidinyl)-ethanon*

Nach der in Beispiel 9 beschriebenen Arbeitsweise erhält man aus 35 g 1-(3-Chlorphenyl)-2-(1-methyl-2-pyrrolidinyliden)-ethanon und 2,83 g Lithiumaluminiumhydrid 21 g der Titelverbindung vom Kp. 112° bei 0,66 Pa.

Beispiel 24

*1-(3-Chlorphenyl)-2-(1-methyl-2-pyrrolidinyl)-ethanol*

Nach der in Beispiel 11 beschriebenen Arbeitsweise erhält man aus 15 g 1-(3-Chlorphenyl)-2-(1-methyl-2-pyrrolidinyl)-ethanon und 1,2 g Natriumborhydrid 12,1 g der Titelverbindung als Diastereomerengemisch vom Kp. 120—125° bei 0,66 Pa.

Beispiel 25

*2-[2-Methoxy-2-(3-methoxyphenyl)-ethyl]-1-methylpyrrolidin*

Zu einer Suspension von 0,25 g 80%igem Natriumhydrid in 15 ml Dimethylformamid tropft man innerhalb 5 Minuten unter Rühren und Feuchtigkeitsausschluß eine Lösung von 1,0 g 1-(3-Methoxyphenyl)-2-(1-methyl-2-pyrrolidinyl)-ethanol in 15 ml Dimethylformamid, rührt anschließend 30 Minuten und tropft eine Lösung von 0,85 g Methyliodid in 10 ml Dimethylformamid zu. Man hält 2 Stunden bei 60°C, versetzt mit Wasser und extrahiert mit Diethylether. Die etherische Phase wird eingeengt und man erhält als Rückstand 0,7 g der Titelverbindung als öliges Diastereomerengemisch.

Beispiel 26

*2-[1-(3-Methoxyphenyl)-2-(1-methylpyrrolidin-2-yl)-ethoxy]-tetrahydropyran*

300 mg (R*,R*)-1-(3-Methoxyphenyl)-2-(1-methyl-2-pyrrolidinyl)-ethanol-hydrochlorid, ein Tropfen etherischer Salzsäure und 3,5 ml 3,4-Dihydro-2H-pyran werden 24 Stunden bei Raumtemperatur gerührt und anschließend zu 390 mg des öligen Hydrochlorids der Titelverbindung eingeengt.

Beispiel 27

*(R*,S*)-Essigsäure-[1-(3-methoxyphenyl)-2-(1-methyl-2-pyrrolidinyl)-ethyl]-ester*

3,5 g (R*,S*)-1-(3-Methoxyphenyl)-2-(1-methyl-2-pyrrolidinyl)-ethanol und 40 ml Essigsäureanhydrid werden 15 Minuten bei Raumtemperatur gerührt. Unter Kühlung wird bis zu einem pH von 8 bis 9 6n Natronlauge zugetropft, die Reaktionsmischung mit Diethylether extrahiert und die organische Phase zur öligen Titelverbindung eingeengt.

Das Hydrochlorid (aus Methanol/Diethylether) schmilzt bei 155—157°.

Beispiel 28

*(R*,R*)-Essigsäure-[1-(3-methoxyphenyl)-2-(1-methyl-2-pyrrolidinyl)-ethyl]-ester*

Nach der in Beispiel 27 beschriebenen Arbeitsweise erhält man aus 3,0 g (R*,R*)-1-(3-Methoxyphenyl)-2-(1-methyl-2-pyrrolidinyl)-ethanol und 30 ml Essigsäureanhydrid 1,56 g der Titelverbindung als zähes, farbloses Öl vom Kp. 130° (1,1 Pa).

Beispiel 29

*1-Pentancarbonsäure-[1-(3-methoxyphenyl)-2-(1-methyl-2-pyrrolidinyl)-ethyl]-ester*

Zu einer Mischung aus 1,17 g 1-(3-Methoxyphenyl)-2-(1-methyl-2-pyrrolidinyl)-ethanol und 0,5 g Triethylamin in 20 ml Dichlormethan tropft man eine Lösung von 0,7 g Hexanoylchlorid in 10 ml Methylenchlorid. Nach 2 Stunden Rühren versetzt man mit 50 ml Wasser, trennt die organische Phase ab, trocknet über Natriumsulfat und engt ein. Man erhält 1,5 g der Titelverbindung als helles, zähes Öl.

Beispiel 30

*1-(3-Methoxyphenyl)-2-(2-pyrrolidinyl)-ethanol*

6,5 g 1-(3-Methoxyphenyl)-2-(2-pyrrolidinyl)-ethanon-hydrochlorid werden in 25 ml Methanol und 5 ml Wasser gelöst und unter Rühren portionsweise mit 4,8 g Natriumborhydrid versetzt. Nach einer Stunde wird die Hauptmenge des Methanols im Vakuum abdestilliert. Der Rückstand wird mit 20 ml Wasser und 10 ml 6n Natronlauge versetzt und mehrmals mit Diethylether extrahiert. Die vereinigten organischen Phasen werden eingeengt und der ölige Rückstand destilliert. Man erhält 4,5 g der Titelverbindung (Diastereomerengemisch) als farbloses Öl vom Kp. 136° (0,66 Pa.).

Beispiel 31

10 000 Tabletten mit einem Wirkstoffgehalt von 30 mg werden aus folgenden Bestandteilen hergestellt:

300 g (R*,R*)-1-(3-Methoxyphenyl)-2-(1-methyl-2-pyrrolidinyl)-ethanol-hydrochlorid (erhalten gemäß Beispiel 13), 800 g Maisstärke, 500 g Milchzucker, 30 g amorphe Kieselsäure und 40 g Natriumlaurylsulfat werden gemischt und gesiebt. Diese Mischung wird mit einer Lösung von 50 g Polyvinylpyrrolidon (mittl. Mol.-Gewicht 25 000) in 320 ml Alkohol befeuchtet und durch ein Sieb mit einer Maschenweite von 1,25 mm granuliert. Das Granulat wird bei 40° getrocknet und mit 160 g Pektin, 100 g Talk und 20 g Magnesiumstearat gemischt. Diese Mischung wird zu Tabletten à 200 mg mit 8 mm Durchmesser verpreßt.

Beispiel 32

*Ansatz für Ampullen*

2 000 g 1-(3-Methoxyphenyl)-2-(1-methyl-2-pyrrolidinyl)-ethanol (erhalten nach Beispiel 11) werden in ca. 80 Liter Aqua bidest unter Zusatz der äquivalenten Menge Salzsäure gelöst, dann werden 400 g Mannit zugegeben. Die Lösung wird auf pH 7,0 $\pm$ 0,5 eingestellt und mit Aqua bidest auf 100 Liter aufgefüllt. Die Lösung wird über ein Filter steril filtriert und unter keimfreien Bedingungen in 2-ml-Ampullen abgefüllt.

Beispiel 33

10 000 Kapseln mit einem Wirkstoffgehalt von 30 mg werden aus folgenden Bestandteilen hergestellt:

3 000 g 1-(3-Methoxyphenyl)-2-(1-methyl-2-pyrrolidinyl)-ethanol (erhalten nach Beispiel 11) werden mit 5 000 g Neutralöl (Miglyol[R]812) gemischt und in Weichgelatinekapseln abgefüllt.

Beispiel 34

*Herstellung einer Charge von 1 000 Suppositorien*

2 350 g kommerzielle Suppositorienmasse werden auf 40—45° erhitzt. In die Schmelze werden 50 g 1-(3-Methoxyphenyl)-2-(1-methyl-2-pyrrolidinyl)-ethanol (erhalten nach Beispiel 11) eingerührt. Die Zäpfchenmasse wird homogenisiert und anschließend in Formen gegossen.

Beispiel 35

*Herstellung einer Charge von 100 Liter Lösung*

10 000 g 1-(3-Methylphenyl)-2-(1-methyl-2-pyrrolidinyl)-ethanol (erhalten nach Beispiel 15) werden unter kräftigem Rühren und Zusatz der äquivalenten Menge Salzsäure in 80 Liter Wasser gegeben, anschließend wird mit 100 g Natriumcyclamat versetzt und mit Wasser auf 100 Liter ergänzt. Die Mischung wird durch eine Korundscheibenmühle geschickt, anschließend entlüftet und dann in 5 ml-Tropfflaschen abgefüllt.

Beispiel 36

Tabletten mit einem Wirkstoffgehalt von 50 mg werden aus folgenden Bestandteilen hergestellt:

25,00 kg (R*,S*)-1-(3-Methoxyphenyl)-2-(1-methyl-2-pyrrolidinyl)-ethanol (erhalten gemäß Beispiel 12), 25,00 kg Indomethacin, 8,50 kg Cellulose (Rehocel[R]), 25,00 kg Milchzucker und 25,00 kg Maisstärke werden gemischt, mit 22,20 kg Polyvinylpyrrolidon (Kollidon[R]25) (in 15 Liter Wasser gelöst) angefeuchtet und granuliert. Danach wird im Trockenschrank bei 50° vorgetrocknet und anschließend durch ein Sieb gegeben. Das Granulat wird bis zu einer relativen Feuchte von 45—50% getrocknet und nach Zugabe von 8,50 kg Carboxymethylcellulose (Primojel), 2,50 kg Talkum und 0,30 kg Magnesiumstearat und sorgfältigem Mischen zu Tabletten von 120 mg Gewicht verpreßt.

Pharmakologie

Die ausgeprägten analgetischen Eigenschaften der erfindungsgemäßen Verbindungen lassen sich an verschiedenen Tierspezies in mehreren Modellversuchen (Hot-plate-Test, Brennstrahl-Test, Writhing-Test) nachweisen, wobei sie sich infolge der geringeren Toxizität und fehlenden Nebenwirkungen (insbesondere Kein Auftreten einer Abhängigkeit oder einer Toleranzentwicklung) handelsüblichen Analgetika überlegen erweisen. Es ist weder eine Abschwächung der Totizität noch eine der analgetischen Wirkung nach wiederholter Gabe nachzuweisen. Auch ist durch Gabe von Naloxon keine Entzugssymptomatik provozierbar.

Der Vergleich der analgetischen Eigenschaften der erfindungsgemäßen Pyrrolidine und denen bekannter starker Analgetika wurde am Beispiel der in Tabelle I angeführten Verbindungen durchgeführt.

Tabelle I

1. Dextropropoxyphen
2. Nefopam
3. 1-(3-Methoxyphenyl)-2-(1-methyl-2-pyrrolidinyl)-ethanol (Beispiel 11)
4. (R*,S*)-1-(3-Methoxyphenyl)-2-(1-methyl-2-pyrrolidinyl)-ethanol (Beispiel 12)
5. (R*,R*)-1-(3-Methoxyphenyl)-2-(1-methyl-2-pyrrolidinyl)-ethanol (Beispiel 13)
6. 1-(3-Methylphenyl)-2-(1-methyl-2-pyrrolidinyl)-ethanol (Beispiel 15)
7. (R*,S*)-1-(3-Methylphenyl)-2-(1-methyl-2-pyrrolidinyl)-ethanol (Beispiel 16)
8. (R*,R*)-1-(3-Methylphenyl)-2-(1-methyl-2-pyrrolidinyl)-ethanol (Beispiel 17)
9. 2-(1-Isopropyl-2-pyrrolidinyl)-1-(3-methoxyphenyl)-ethanol (Beispiel 14)
10. 1-(3-Hydroxyphenyl)-2-(1-methyl-2-pyrrolidinyl)-ethanol (Beispeil 19)
11. 1-(3-Chlorphenyl)-2-(1-methyl-2-pyrrolidinyl)-ethanol (Beispiel 24)
12. 2-(1-n-Hexyl-2-pyrrolidinyl)-1-(3-methoxyphenyl)-ethanol (Beispiel 22)
13. (R*,S*)-Essigsäure-[1-(3-methoxyphenyl)-2-(1-methyl-2-pyrrolydinyl)-ethyl]-ester (Beispiel 27)

In der Tabelle II werden die ermittelten $LD_{50}$-Werte nach oraler Gabe sowie die in den verschiedenen Analgesietests nach oraler Applikation erhaltenen $ED_{50}$-Werte (mg/kg) wiedergegeben.

TABELLE II

Vergleich der Toxizität $LD_{50}$ [mg/kg, p.o.] und der analgetischen Wirkung [$ED_{50}$-Werte (mg/kg, p.o.)] von bekannten Analgetika und substituierten Pyrrolidinen

| Verbindung | $LD_{50}$ | $ED_{50}$ | | | |
|---|---|---|---|---|---|
| | | Maus | | | |
| lfd. Nr. | Maus | Hot-plate | Brennstrahl | Writhing | Ratte Brennstrahl |
| 1 | 140 | 15 | 25 | 30 | 25 |
| 2 | 180 | 30 | 20 | 25 | 10 |
| 3 | 600 | 30 | 25 | 15 | 7 |
| 4 | 420 | 100 | 60 | 50 | – |
| 5 | 490 | 50 | 70 | 40 | – |
| 6 | 420 | 100 | 35 | 75 | 10 |
| 7 | 300 | 100 | 50 | 75* | – |
| 8 | 320 | 100* | 10 | 75* | – |
| 9 | 340 | 100* | 75* | 75 | – |
| 10 | 420 | – | – | 125 | – |
| 11 | 330 | – | 65 | 125 | – |
| 12 | 180 | – | 70 | 75* | – |
| 13 | 440 | – | – | 75* | – |

*) $ED_{40}$-Werte.

Nach Way et al. [J.Pharmacol.Exp.Ther.167(1969)1] reagieren Mäuse, die von morphinartig wirkenden Analgetika abhängig sind, unmittelbar nach i.p. Gaben von Naloxon mit Entzugsspringen (jumping). Nach Saelens und Mitarbeitern [Arch.Int.Pharmacodyn.Ther.190(1971)213]läßt sich auch für 1 eine Abhängigkeitsentwicklung nachweisen, 2 ist in dieser Form aufgrund der Toxizität nicht prüfbar.

3 bzw. 6 wurden an Mäuse appliziert [2 mal täglich 200 mg/kg über 5 Wochentage]. Die i.p. Applikation von 30 mg/kg Naloxon 1 Stunde nach Gabe der letzten Dosis der erfindungsgemäßen Verbindungen führte bei den mit den erfindungsgemäßen Verbindungen behandelten Tieren nicht zum Entzugsspringen (siehe Tabelle III).

TABELLE III

"Jumping" von Mäusen nach verschiedener Vorbehandlung

| Behandlung | % der Tiere, die nach i.p. Gabe von Naloxon sogenannte jumpings zeigen |
|---|---|
| NaCl 0,9 %ig | 20 % |
| 1 | 100 % |
| 2 | aufgrund seiner Toxizität nicht prüfbar Letalität: 80 % |
| 3 | 20 % |
| 6 | 10 % |

Teste mit den substituierten Pyrrolidinen an Maus und Ratte im subtoxischen Dosisbereich (Reserpin-Antagonismus, Haloperidol-Antagonismus, Hexobarbital-Narkose, Perphenazin- und Tetrabenazin-Antagonismus) ergeben keinen Hinweis auf spezifische Nebenwirkungen.

Die Ermittlung der pharmakologischen Eigenschaften erfolgte nach folgenden Methoden:

*Analgesie*

a) Hot-plate-Test: Weibliche Albinomäuse werden auf eine 50°C heiße Platte gesetzt und die Reaktionszeit bis zum Pfotenlecken gestoppt. Normalwerte liegen im Bereich von 7—8 Sekunden. Die geprüften Substanzen bewirken verzögerte Reaktion auf den Wärmereiz, d.h. eine verminderte Wärmeschmerzempfindlichkeit. Bestimmt wurde die Dosis, die die Reaktionszeit um 50% verlängert. Literatur: Eddy, N.B. and Leimbach, D. (1953) J.Pharmacol.Exp.Ther.107,385.

b) Brennstrahl-Test: Weiblichen Albinomäusen bzw. Ratten wird mit einem focussierten Wärmestrahl ein thermischer Schmerz an der Schwanzwurzel gesetzt und die Zeit bis zum Wegziehen des Schwanzes gestoppt. Normalerweise liegt sie im Bereich von 4—5 Sekunden. Die geprüften Substanzen bewirken verzögerte Reaktion auf den Wärmeschmerz, d.h. eine verminderte Wärmeschmerzreaktion. Bestimmt wurde die Dosis, die die Reaktionszeit um 50% verlängert. Literatur: D'Amour, F.E. and Smith D.L. (1941) J.Pharmacol.Exp.Ther.72,74.

c) Writhing Test (Essigsäure-Writhing): Intraperitoneale Injektion von 0,2 ml/20 g Maus einer 0,75%igen Essigsäurelösung induziert bei Albinomäusen ein typisches über den Körper mit Dorsalflektion ablaufendes Syndrom, "Writhing" genannt. Diese im Verlauf der ersten halben Stunde p.a. auftretenden "Writhings" werden im Zeitraum von 5—20 Minuten p.a. gezählt. Die geprüften Substanzen bewirken eine Verminderung der Anzahl der Writhing-Syndrome. Bestimmt wurde die Dosis, die deren Anzahl, bezogen auf die Tageskontrolle, um 50% reduziert. Literatur: Koster, Anderson, de Beer (1959) Fed.Proc. 18, 412.

*Bestimmung der letalen Wirkung*

Die Toxizitätsuntersuchungen wurden an weiblichen NMRI-Mäusen, Körpergewicht 22—26 g, durchgeführt. Die Tiere erhielten Futter und Wasser (Altromin$^R$) ad libitum. Die zu prüfenden Substanzen wurden als Lösungen verschiedener Konzentration mit der Schlundsonde in einem Volumen von 10—20 ml/kg oral verabreicht; 5 Tiere pro Dosis wurden in Makrolonkäfigen, Typ II, gehalten. Die Beobachtungsdauer betrug 48 Stunden. Die $DL_{50}$, die Dosis, bei der 50% der Tiere starben, wurde aus der Dosiswirkungskurve graphisch ermittelt.

**Patentansprüche**

1. Substituierte Pyrrolidine der allgemeinen Formel I

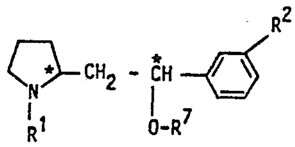

(I),

worin

$R^1$ ein Wasserstoffatom oder eine Alkylgruppe mit 1 bis 7 Kohlenstoffatomen,

$R^2$ ein Chloratom, eine Hydroxygruppe, eine Alkylgruppe mit 1 bis 4 Kohlenstoffatomen oder eine Alkoxygruppe mit 1 bis 4 Kohlenstoffatomen und

$R^7$ ein Wasserstoffatom, eine Alkanoylgruppe mit 1 bis 11 Kohlenstoffatomen, eine Alkylgruppe mit 1 bis 7 Kohlenstoffatomen oder eine Tetrahydropyranylgruppe bedeuten,

und ihre Säureadditionssalze.

2. Substituierte Pyrrolidine der allgemeinen Formel I*

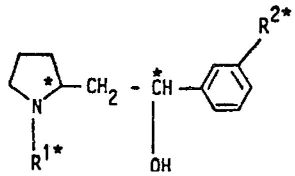

(I*),

worin

$R^{1*}$ ein Wasserstoffatom oder eine Alkylgruppe mit 1 bis 3 Kohlenstoffatomen und

$R^{2*}$ eine Hydroxygruppe, eine Methylgruppe oder eine Methoxygruppe bedeuten,

und ihre Säureadditionssalze.

3. Substituierte Pyrrolidine der allgemeinen Formel I**

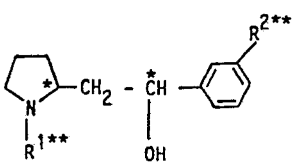

(I**),

worin

$R^{1**}$ ein Wasserstoffatom oder eine Methylgruppe und

$R^{2**}$ eine Methylgruppe oder eine Methoxygruppe bedeuten,

und ihre pharmakologisch verträglichen Säureadditionssalze.

4. 2-(1-Methylpyrrolidin-2-yl)-1-(m-tolyl)-ethanol und seine Säureadditionssalze.

5. 1-(3-Methoxyphenyl)-2-(1-methylpyrrolidin-2-yl)-ethanol und seine Säureadditionssalze.

6. Verbindungen gemäß Anspruch 1 bis 5 in Form der Diastereomeren, Enantiomeren, Racemate oder ihrer Gemische.

7. Arzneimittel enthaltend eine oder mehrere Verbindungen gemäß Anspruch 1 bis 6.

8. Verfahren zur Herstellung der substituierten Pyrrolidine der allgemeinen Formel I sowie der Säureadditionssalze, dadurch gekennzeichnet, daß man ein Keton der allgemeinen Formel II oder III

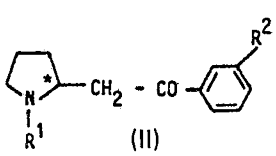

(II)

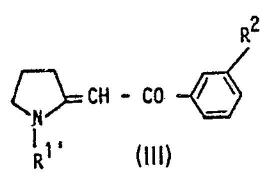

(III)

worin

$R^1$ ein Wasserstoffatom oder eine Alkylgruppe mit 1 bis 7 Kohlenstoffatomen und

$R^2$ ein Chloratom, eine Hydroxygruppe, eine Alkylgruppe mit 1 bis 4 Kohlenstoffatomen oder eine Alkoxygruppe mit 1 bis 4 Kohlenstoffatomen bedeuten,

$R^{1'}$ eine Alkylgruppe mit 1 bis 7 Kohlenstoffatomen bedeutet,

oder dessen Säureadditionssalz reduziert und gegebenenfalls anschließend acyliert oder verethert und/oder die erhaltene freie Base oder ihr Säureadditionssalz ineinander überführt.

9. Verfahren zur Herstellung der substituierten Pyrrolidine der allgemeinen Formel I* sowie der Säureadditionssalze, dadurch gekennzeichnet, daß man ein Keton der allgemeinen Formel II* oder III*

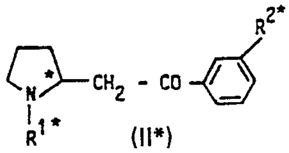
(II*)

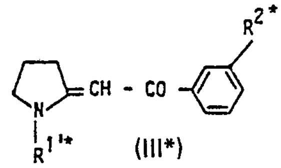
(III*)

worin

$R^{1*}$ eine Wasserstoffatom oder eine Alkylgruppe mit 1 bis 3 Kohlenstoffatomen und

$R^{2*}$ eine Hydroxygruppe, eine Methylgruppe oder eine Methoxygruppe bedeuten, und

$R^{1'*}$ eine Alkylgruppe mit 1 bis 3 Kohlenstoffatomen bedeutet,

oder dessen Säureadditionssalz reduziert und gegebenenfalls anschließend die erhaltene freie Base oder ihr Säureadditionssalz ineinander überführt.

10. Verfahren zur Herstellung der substituierten Pyrrolidine der allgemeinen Formel I** sowie der Säureadditionssalze, dadurch gekennzeichnet, daß man ein Keton der allgemeinen Formel II** oder III**

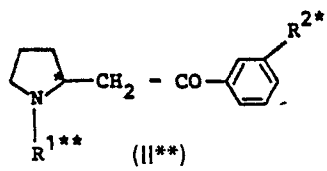
(II**)

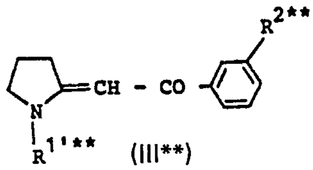
(III**)

worin

$R^{1**}$ ein Wasserstoffatom oder eine Methylgruppe,

$R^{2**}$ eine Methylgruppe oder eine Methoxygruppe und

$R^{1'**}$ eine Methylgruppe bedeuten,

oder dessen Säureadditionssalz reduziert und gegebenenfalls anschließend die erhaltene freie Base oder ihr Säureadditionssalz ineinander überführt.

11. Verfahren gemäß Anspruch 8, 9 oder 10, dadurch gekennzeichnet, daß die Reduktion der Verbindungen III, III* oder III** zweistufig durchgeführt wird.

12. Verbindungen gemäß Anspruch 1 bis 6 zur Verwendung bei der Bekämpfung von Schmerzzuständen.

**Revendications**

1. Pyrrolidines substituées de la formule générale I

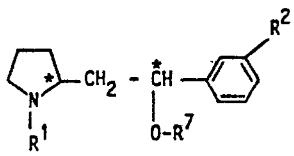
(I)

dans laquelle

$R^1$ représente un atome d'hydrogène ou un groupe alkyle comportant de 1 à 7 atomes de carbone,

$R^2$ représente un atome de chlore, un groupe hydroxy, un groupe alkyle comportant de 1 à 4 atomes de carbone ou un groupe alcoxy comportant de 1 à 4 atomes de carbone, et

$R^7$ représente un atome d'hydrogène, un groupe alcanoyle comportant de 1 à 11 atomes de carbone, un groupe alkyle comportant de 1 à 7 atomes de carbone ou un groupe tétrahydropyranyle, et leurs sels d'addition avec des acides.

2. Pyrrolidines substituées de la formule générale I*

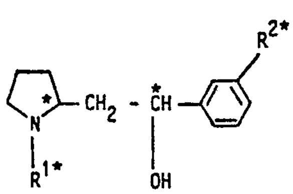
(I*)

dans laquelle

$R^{1*}$ représente un atome d'hydrogène ou un groupe alkyle comportant de 1 à 3 atomes de carbone, et

$R^{2*}$ représente une groupe hydroxy, un groupe méthyle ou un groupe méthoxy. et leurs sels d'addition avec des acides.

3. Pyrrolidines substituées de la formule générale I**

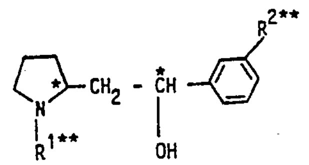

(I**)

dans laquelle

$R^{1**}$ représente un atome d'hydrogène ou un groupe méthyle, et

$R^{2**}$ représente un groupe méthyle ou un groupe méthoxy,

et leurs sels d'addition pharmacologiquement compatibles avec des acides.

4. 2-(1-Méthylpyrrolidin-2-yl)-1-(m-tolyl)-éthanol et ses sels d'addition avec des acides.

5. 1-(3-Méthoxyphényl)-2-(1-méthylpyrrolidin-2-yl)-éthanol et ses sels d'addition avec des acides.

6. Composés selon les Revendications 1 à 5, caractérisés en ce qu'ils sont sous forme de diastéréomères, d'énantiomères, de racémates ou des mélanges de ceux-ci.

7. Médicaments caractérisés en ce qu'ils contiennent un ou plusieurs des composés selon les Revendications 1 à 6.

8. Procédé pour la préparation de pyrrolidines substituées de la formule générale I, ainsi que de leurs sels d'addition avec des acides, caractérisé en ce qu'on réduit une cétone de la formula générale II ou de la formula générale III

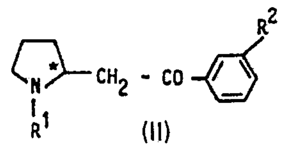

(II)

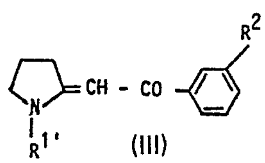

(III)

dans lesquelles

$R^1$ représente un atome d'hydrogène ou un groupe alkyle comportant de 1 à 7 atomes de carbone,

$R^2$ représente un atome de chlore, un groupe hydroxy, un groupe alkyle comportant de 1 à 4 atomes de carbone ou un groupe alcoxy comportant de 1 à 4 atomes de carbone, et

$R^{1'}$ représente un groupe alkyle comportant de 1 à 7 atomes de carbone,

ou bien l'on réduit un sel d'addition avec un acide de ces composés, après quoi l'on acyle ou éthérifie, le cas échéant, le composé obtenu et/ou l'on transforme, le cas échéant, la base libre obtenue ou le sel d'addition avec un acide obtenu, l'un dans l'autre.

9. Procédé de préparation de pyrrolidines substituées de la formule générale I* ainsi que le leurs sels d'addition avec des acides, caractérisé en ce qu'on réduit une cétone de la formule générale II* ou de la formule générale III*

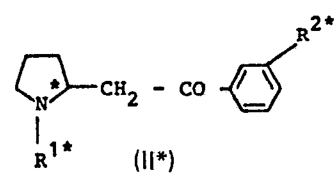

(II*)

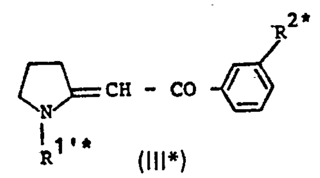

(III*)

dans lesquelles

$R^{1*}$ représente un atome d'hydrogène ou un groupe alkyle comportant de 1 à 3 atomes de carbone,

$R^{2*}$ représente un groupe hydroxy, un groupe méthyle ou un groupe méthoxy, et

$R^{1'*}$ représente un groupe alkyle comportant de 1 à 3 atomes de carbone,

ou bien l'on réduit un sel d'addition avec un acide d'un composé de la formule II* ou III*, après quoi l'on transforme, le cas échéant, la base libre obtenue ou le sel d'addition avec un acide obtenue, l'un dans l'autre.

10. Procédé de préparation de pyrrolidines substituées de la formule générale I** ainsi que de leurs sels d'addition avec des acides, caractérisé en ce qu'on réduit une cétone de la formule générale II** our de la formule générale III**

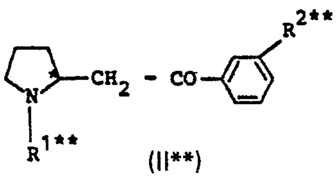

(II**)

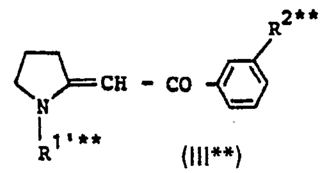

(III**)

dans lesquelles

R[1**] représente un atome d'hydrogène ou un groupe méthyle,

R[2**] représente un groupe méthyle ou un groupe méthoxy, et

R[1'**] représente un groupe méthyle,

ou on réduit un sel d'addition avec un acide de ces composés de la formule II** ou III**, après quoi l'on transforme, le cas échéant, la base libre obtenue ou le sel d'addition avec un acide obtenu, l'un dans l'autre.

11. Procédé selon la Revendication 8, 9 ou 10, caractérisé en ce que la réduction des composés III, III* ou III** est réalisée en deux étapes.

12. Composés selon les Revendications 1 à 6 pour l'utilisation pour le traitement d'états douloureux.

## Claims

1. Substituted pyrrolidines of the general formula I

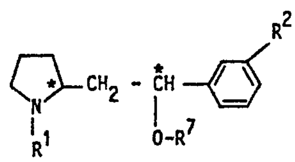

(I)

wherein

$R^1$ denotes a hydrogen atom or an alkyl group with 1 to 7 carbon atoms,

$R^2$ denotes a chlorine atom, a hydroxyl group, an alkyl group with 1 to 4 carbon atoms or an alkoxy group with 1 to 4 carbon atoms, and

$R^7$ denotes a hydrogen atom, an alkanoyl group with 1 to 11 carbon atoms, an alkyl group with 1 to 7 carbon atoms or a tetrahydropyranyl group,

and their acid addition salts.

2. Substituted pyrrolidines of the general formula I*

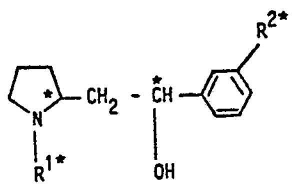

(I*)

wherein

$R^{1*}$ denotes a hydrogen atom or an alkyl group with 1 to 3 carbon atoms, and

$R^{2*}$ denotes a hydroxyl group, a methyl group or a methoxy group,

and their acid addition salts.

3. Substituted pyrrolidines of the general formula I**

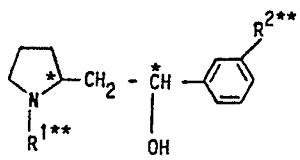

(I**)

wherein

$R^{1**}$ denotes a hydrogen atom or a methyl group, and

$R^{2**}$ denotes a methyl group or a methoxy group,

and their pharmacologically compatible acid addition salts.

4. 2-(1-methylpyrrolidin-2-yl)-1-(m-tolyl)-ethanol and its acid addition salts.

5. 1-(3-methoxyphenyl)-2-(1-methylpyrrolidin-2-yl)-ethanol and its acid addition salts.

6. Compounds according to Claim 1 to 5 in the form of the diastereoisomers, enantiomers, racemates or their mixtures.

7. Medicaments containing one or more compounds according to Claim 1 to 6.

8. Process for the preparation of the substituted pyrrolidines of the general formula I and the acid addition salts, characterised in that a ketone of the general formula II or III

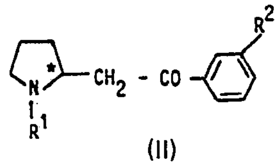
(II)

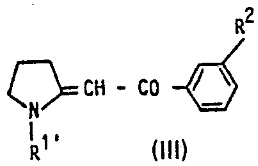
(III)

wherein

$R^1$ denotes a hydrogen atom or an alkyl group with 1 to 7 carbon atoms, and

$R^2$ denotes a chlorine atom, a hydroxyl group, an alkyl group with 1 to 4 carbon atoms or an alkoxy group with 1 to 4 carbon atoms,

$R^{1'}$ denotes an alkyl group with 1 to 7 carbon atoms, or its acid addition salt, is reduced and, where appropriate, subsequently acylated or etherified and/or the free base obtained is converted into its acid addition salt or the acid addition salt obtained is converted into the free base.

9. Process for the preparation of the substituted pyrrolidines of the general formula I* and of the acid addition salts, characterised in that a ketone of the general formula II* or III*

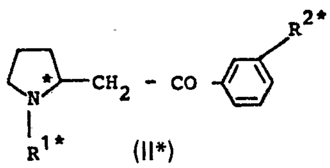
(II*)

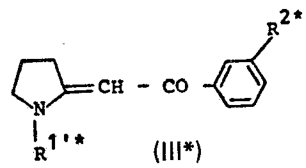
(III*)

wherein

$R^{1*}$ denotes a hydrogen atom or an alkyl group with 1 to 3 carbon atoms, and

$R^{2*}$ denotes a hydroxyl group, a methyl group or a methoxy group, and

$R^{1'*}$ denotes an alkyl group with 1 to 3 carbon atoms, or its addition salt, is reduced and, where appropriate the free base obtained is converted into its acid addition salt or the acid addition salt obtained is converted into the free base.

10. Process for the preparation of the substituted pyrrolidines of the general formula I** and of the acid addition salts, characterised in that a ketone of the general formula II** or III**

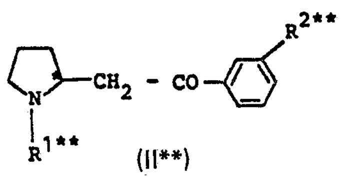
(II**)

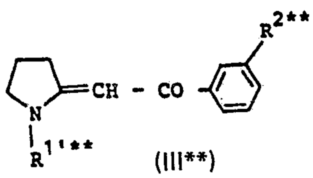
(III**)

wherein

$R^{1**}$ denotes a hydrogen atom or a methyl group,

$R^{2**}$ denotes a methyl group or a methoxy group, and

$R^{1'**}$ denotes a methyl group,

or its acid addition salt, is reduced and, where appropriate, the free base obtained is converted into its acid addition salt or the acid addition salt obtained is converted into the free base.

11. Process according to Claim 8, 9 or 10, characterised in that the reduction of the compounds III, III* or III** is carried out in two steps.

12. Compounds according to Claim 1 to 6 for use in combating pain conditions.